# EUROPÄISCHE PATENTSCHRIFT

(11) **EP 2 183 600 B1**
(45) Veröffentlichungstag und Bekanntmachung des Hinweises auf die Patenterteilung: **03.09.2014**
(21) Anmeldenummer: 08784332.2
(22) Anmeldetag: 10.07.2008
(51) Int. Cl.: G01N 33/68

(54) **VERWENDUNG VON CYANOZIMTSÄUREDERIVATEN ALS MATRIZES IN MALDI- MASSENSPEKTROMETRIE**
USE OF CYANOCINNAMIC ACID DERIVATIVES AS MATRICES IN MALDI MASS SPECTROMETRY
UTILISATION DE DÉRIVÉS D'ACIDE CYANOCINNAMIQUE EN TANT QUE MATRICES DANS LE CADRE D'UNE SPECTROMÉTRIE DE MASSE MALDI

(30) Priorität: 27.08.2007 DE 102007040251
(43) Veröffentlichungstag der Anmeldung: 12.05.2010
(73) Patentinhaber: Sigma-Aldrich International GmbH, 9000 St. Gallen (CH)
(72) Erfinder: KARAS, Michael, 65795 Hattersheim (DE); JASKOLLA, Thorsten, 64331 Weiterstadt (DE)
(74) Vertreter: Scholz, Volker
(86) Internationale Anmeldenummer: PCT/DE2008/001149
(87) Internationale Veröffentlichungsnummer: WO 2009/026867

(56) Entgegenhaltungen:
- WO-A-2006/124003
- BEAVIS R C ET AL: "Cinnamic acid derivatives as matrices for ultraviolet laser desorption mass spectrometry of proteins." RAPID COMMUNICATIONS IN MASS SPECTROMETRY : RCM DEC 1989, Bd. 3, Nr. 12, Dezember 1989 (1989-12), Seiten 432-435, XP009017328 ISSN: 0951-4198
- THOLEY ANDREAS ET AL: "Ionic (liquid) matrices for matrix-assisted laser desorption/ionization mass spectrometry-applications and perspectives." ANALYTICAL AND BIOANALYTICAL CHEMISTRY SEP 2006, Bd. 386, Nr. 1, September 2006 (2006-09), Seiten 24-37, XP002504011 ISSN: 1618-2642
- JASKOLLA THORSTEN W ET AL: "4-Chloro-alpha-cyanocinnamic acid is an advanced, rationally designed MALDI matrix" PROCEEDINGS OF THE NATIONAL ACADEMY OF SCIENCES OF THE UNITED STATES OF AMERICA, Bd. 105, Nr. 34, August 2008 (2008-08), Seiten 12200-12205, XP002504012 ISSN: 0027-8424

## Beschreibung

Die vorliegende Erfindung betrifft die Verwendung von Cyanozimtsäurederivaten als Matrix in der MALDI-Massenspektrometrie von Analyten.

MALDI (Matrix Assisted Laser Desorption/Ionization, Matrix-unterstützte Laser-Desorption/Ionisierung) ist ein Verfahren zur Ionisierung von Molekülen. Es hat sich seit seiner Entwicklung in den 1980er Jahren als besonders effektiv für die Massenspektrometrie an großen Molekülen und Polymeren sowie Biopolymeren erwiesen. MALDI beruht auf der Co-kristallisation einer Matrix und eines Analyten mit einem deutlichen molaren Überschuß an Matrix. Als Matrixsubstanzen werden dabei kleine organische Moleküle gewählt, die bei der verwendeten Laserwellenlänge Energie absorbieren. Die Matrix erleichtert in großem Maße die Erzeugung von intakten Gasphasenionen aus großen, nicht-flüchtigen und thermisch labilen Verbindungen, wie Peptiden, Proteinen, Oligonukleotiden, synthetischen Polymeren und großen anorganischen Verbindungen. Die Matrizes können praktisch für alle mit MALDI-MS analysierbaren Substanzen eingesetzt werden, also für große und kleine, nicht flüchtige und thermisch labile Verbindungen, wie Biomakromoleküle, wie Proteine und Lipide, sowie kleine, organische und anorganische Analyte, wie Arzneistoffe, Pflanzenmetabolite und dergleichen. Das Haupteinsatzgebiet liegt bei der Analyse von Peptiden. Ein Laserstrahl (UV- oder IR-gepulster Laser) dient als die Energiequelle zur Desorption und Ionisierung.

Die Matrix spielt eine Schlüsselrolle, indem sie die Laserlichtenergie absorbiert und im angeregten Zustand einen kleinen Teil der Matrix-Analyt-Cokristalle ablatiert, wodurch Probenmoleküle in die Gasphase gelangen. Durch Wechselwirkung zwischen Matrix und Analyt entstehen Analytionen, die nach Bildung elektrostatisch in ein Massenspektrometer überführt werden, in dem sie von den Matrixionen getrennt und einzeln detektiert werden können.

Als Matrixsubstanzen werden bislang kleine organische Moleküle gewählt, beispielsweise Verbindungen mit labilen Protonen, wie Carbonsäuren. Die bekannteste Matrixverbindung ist die überwiegend eingesetzte α-Cyano-4-hydroxyzimtsäure (CHCA oder HCCA). Weitere in der MALDI-MS Verwendung findende Matrizes sind zum Beispiel Sinapinsäure (4-Hydroxy-3,5-dimethoxyzimtsäure) oder 2,5-Dihydroxybenzoesäure (2,5-DHB).

Aus Beavis, R.C.; Chait, B.T.: Cinnamic Acid Derivatives as Matrices for Ultraviolet Laser Desorption Mass Spectrometry of Proteins. Rapid Communication in Mass Spectrometry (1989), Vol. 3, Nr. 12, Seiten 432-435 ist die Verwendung von hydroxy- bzw. methoxysubstituierten Zimtsäuren als Matrizes in der MALDI-Massenspektrometrie bekannt. Aus US 2002/0142982 A1 geht die entsprechende Verwendung von α-Cyanozimtsäure hervor. DE 101 58 860 A1 und DE 103 22 701 A1 beschreiben die Verwendung von α-Cyano-4-hydroxyzimtsäure bzw. 3,5-Dimethoxy-4-hydroxyzimtsäure. Schließlich offenbaren US 2006/0040334 A1 und WO 2006/124003 A1 MALDI-Matrizes auf der Basis von analytgekoppelten oder polymer-gekoppelten Derivaten der α-Cyano-4-hydroxyzimtsäure.

Die für die MALDI-Massenspektrometrie einsetzbaren Matrizes müssen gleichzeitig eine große Anzahl von Voraussetzungen erfüllen. Zunächst müssen sie in der Lage sein, Analyte (durch Co-Kristallisation) in den Matrixkristall einzubauen und von anderen Analyten zu isolieren. Ferner müssen sie in Lösungsmitteln, die mit dem Analyt kompatibel sind, löslich sein, vakuumstabil sein und die Laserwellenlänge des eingesetzten Lasers absorbieren. Ferner sollen sie eine Co-Desorption von Matrix und Analyt bei Laserbestrahlung verursachen und eine Analytionisierung fördern.

Für die bislang eingesetzten Matrixverbindungen sind die Eigenschaften und damit die Leistungsdaten der MALDI-Massenspektrometrie insbesondere im Hinblick auf die Detektion und Nachweisempfindlichkeit für positive Ionen, negative Ionen bzw. zweifach und höher geladene Ionen noch verbesserungsfähig.

Es ist daher eine Aufgabe der vorliegenden Erfindung, Verbindungen bereitzustellen, die als Matrix in der MALDI-Massenspektrometrie verwendet werden können und die Nachteile aus dem Stand der Technik zumindest teilweise überwinden. Insbesondere soll Detektion und Nachweisempfindlichkeit für positive Ionen, negative Ionen und zweifach und höher geladene Ionen verbessert werden.

Diese Aufgabe wird gelöst durch die Verwendung von Cyanozimtsäurederivaten der allgemeinen Formel: 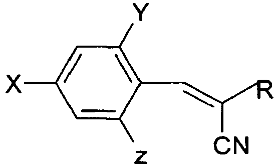 wobei X ausgewählt wird aus CI, F, Br, I und substituiertem und unsubstituiertem C₁-C₁₀-Alkyl; Y und Z unabhängig ausgewählt werden aus H, Cl, F, Br, I und substituiertem und unsubstituiertem C₁-C₁₀-Alkyl; und R ausgewählt wird aus COOH, CONH₂, SO₃H und COOR' mit R' = C₁-C₁₀-Alkyl als Matrix für eine MALDI-Massenspektrometrie eines Analyten. Die gezeigte Strukturformel soll alle möglichen cis/trans-Isomere der Cyanozimtsäurederivate einschließen.

Bevorzugt ist dabei, dass X ausgewählt wird aus F, Cl, Methyl oder C₂-C₁₀-Alkyl und Y und Z unabhängig ausgewählt werden aus H, F und Cl.

Auch wird vorgeschlagen, dass das Cyanozimtsäurederivat 2,4-Difluor-cyanozimtsäure, 4-Chlor-cyanozimtsäure oder 4-Methyl-cyanozimtsäure ist.

In einer bevorzugten Ausführungsform ist 4-Chlor-cyanozimtsäure die Matrix zur MALDI-Massenspektrometrie positiver Ionen.

In einer weiteren bevorzugten Ausführungsform sind 2,4-Difluor-cyanozimtsäure, 4-Chlor-cyanozimtsäure und 4-Methyl-cyanozimtsäure Matrizes zur MALDI-Massenspektrometrie negativer Ionen.

Des Weiteren wird vorgeschlagen, dass 2,4-Difluor-cyanozimtsäure die Matrix zur MALDI-Massenspektrometrie negativer und positiver Ionen ist.

In noch einer weiteren Ausführungsform sind 4-Methyl-cyanozimtsäure und 4-Chlor-cyanozimtsäure Matrizes zur MALDI-Massenspektrometrie zweifach geladener Ionen.

Auch wird bevorzugt, dass das Analyt ein verdautes Protein oder Peptid ist. Jedoch sind auch unverdaute Proteine, Polynukleinsäuren, Saccharide und dergleichen als Analyt denkbar.

Bevorzugt ist auch, dass die Matrix mit dem Analyten vermischt wird.

Ferner wird vorgeschlagen, dass das molare Mischungsverhältnis von Analyt zu Matrix 1:1000 bis 1:1000000000, bevorzugt 1:100000-1:10000000 beträgt.

Auch ist bevorzugt vorgesehen, dass Mischungen unterschiedlicher unter obige allgemeine Formel fallende Cyanozimtsäure-Derivate oder eine Mischung zumindest eines unter obige allgemeine Formel fallenden Cyanozimtsäure-Derivats mit einer weiteren Matrix eingesetzt werden bzw. wird.

Schließlich wird vorgeschlagen, dass die weitere Matrix ausgewählt wird aus α-Cyano-4-hydroxyzimtsäure, 2,5-Dihydroxybenzoesäure oder Sinapin- oder Ferulasäure.

Ebenfalls können 2-Aza-5-thiothymin oder 3-Hydroxypicolinsäure als weitere Matrix eingesetzt werden.

Ebenfalls ist denkbar, dass die eingesetzten Matrixmaterialien mit einem inerten Füllstoff vermischt werden.

Überraschenderweise wurde erfindungsgemäß festgestellt, dass bei Einsatz der oben aufgeführten Cyanozimtsäurederivate als Matrix in der MALDI-Massenspektrometrie die Detektion und Nachweisempfindlichkeit für positive Ionen, negative Ionen und zweifach und höher geladene Ionen deutlich verbessert werden kann.

Insbesondere wurde eine deutliche Steigerung der Nachweisempfindlichkeit von Biopolymeranalyten, vorzugsweise Peptidanalyten, erzielt. Ferner zeigt die erfindungsgemäße Verwendung eine geringe Selektivität für Peptide verschiedener Aminosäurezusammensetzungen und damit deutlich geringere Diskriminierungseffekte in den MALDI-Massenspektren als bei den bisherigen verwendeten Matrizes, wodurch in einem Analytgemisch mehr Analytarten detektiert werden können als es bisher der Fall war. Ein Einsatz von Matrix-Mischungen der oben beschriebenen Cyanozimtsäurederivaten erlaubt eine Optimierung der Eigenschaften im Hinblick auf die Kristallisation und den Einbau von Analytverbindungen in die Matrixkristalle sowie eine effizientere Ionisation der Analyte.

Überraschenderweise wurde ebenfalls festgestellt, dass Cyanozimtsäurederivate mit Substituenten mit ähnlichen elektronischen Eigenschaften, wie NO₂, CN oder Acetyl, nicht annähernd so gute Ergebnisse erzielen wie die erfindungsgemäß verwendeten Cyanozimtsäurederivate.

Weitere Merkmale und Vorteile der Erfindung ergeben sich aus der folgenden detaillierten Beschreibung bevorzugter Ausführungsformen in Verbindung mit den beigefügten Zeichnungen, in denen
Figuren 1-5 Massenspektrenausschnitte eines BSA-Proteinverdaus (BSA: Bovine Serum Albumin, Rinderserumalbumin) zeigen, in einem Vergleich bei Verwendung der bislang eingesetzten α-Cyano-4-hydroxyzimtsäure und der neu verwendeten α-Cyano-4-chlorzimtsäure als Matrix;
Figur 6 in Form einer Tabelle alle detektierbaren Peptide eines BSA-Proteinverdaus aufführt;
Figur 7 einen Vergleich von Massenspektren für negative Ionen der α-Cyano-4-hydroxyzimtsäure und α-Cyano-2,4-difluorzimtsäure als Matrix zeigt; und
Figur 8 Massenspektren für doppelt geladene Ionen unter Verwendung von α-Cyano-4-hydroxyzimtsäure und α-Cyano-4-methylzimtsäure zeigt.

Erfindungsgemäß einsetzbare Cyanozimtsäurederivate können beispielsweise durch Kondensation des entsprechenden Aldehyds mit Cyanoessigsäure(derivaten) gemäß einer Knoevenagel-Kondensation hergestellt werden.

### Beispiel 1

2 g Cyanessigsäure (1 Äq., 23,5 mmol), 2,97 g Chlorbenzaldehyd (0,9 Äq., 21,1 mmol) und 300 mg Ammoniumacetat (15% (m/m) bezogen auf die Säure) werden in 30 ml Toluol unter Verwendung eines Wasserabscheiders unter Rückfluß erwärmt. Nach beendeter Wasserabscheidung (etwa 3 Stunden) wird auf Raumtemperatur abgekühlt, wobei das Produkt üblicherweise kristallin ausfällt. Nach Filtration wird das Rohprodukt mit ausreichend Wasser gewaschen. Erfolgt kein Produktausfall, so wird eingeengt und der verbliebene Feststoff mit ausreichend Wasser gewaschen. Das Rohprodukt wird wiederholt aus einem Alkohol (Methanol)/Wasser-Gemisch umkristallisiert. Weitergehende Aufreinigung erfolgt durch Ionenaustausch (starker Kationenaustauscher), wobei als Lösungsmittel 50% Acetonitril verwendet wird. Das Eluat wird bei Raumtemperatur eingeengt, wobei das aufgereinigte Produkt kristallin ausfällt. Nach Abfiltrieren und Trocknen im Vakuum erhält man 3,35 g (78% der Theorie) α-Cyano-4-chlorzimtsäure.

### Beispiel 2

Die bezogen auf die Aldehydstoffmenge 1,25-fache Stoffmenge an Cyanessigsäure wird in einem Becherglas zusammen mit der bezogen auf die Cyanessigsäure 1,1-fachen Stoffmenge an Soda bei 30°C in 40 ml Wasser gelöst. Die Lösung wird in einen Kolben überführt und die anschließende Reaktion bei etwa 30°C durchgeführt. Zu der Lösung wird Natriumhydroxid (0,125-fache Stoffmenge bezüglich der Cyanessigsäurestoffmenge) gelöst in 15 ml Wasser hinzugegeben. Das Aldehyd wird - sofern nicht flüssig - im kleinstmöglichen Volumen Acetonitril gelöst und über einen Zeitraum von etwa 30 Minuten unter starkem Rühren allmählich zugegeben. Nach Rühren für 2 Stunden wird die Lösung in ein Becherglas überführt, mit konzentrierter Salzsäure angesäuert, die Lösung im Eisbad abgekühlt und abgenutscht. Das Präzipitat wird mit wenig kaltem Wasser und Toluol (sofern unlöslich) gewaschen und im Vakuum bei 40°C getrocknet. Eine Aufreinigung erfolgt wie in Beispiel 1. Die Ausbeuten betragen etwa 50-70% der Theorie.

Die Cyanozimtsäurederivate können durch übliche Verfahren mit den Analyten vermischt werden, um eine geeignete Probe für die MALDI-Massenspektrometrie bereitzustellen.

Ein beispielhaftes Verfahren zur Vermischung ist beispielsweise das Dried Droplet-Verfahren. Dabei werden Matrix und Analyt gelöst und zeitgleich (durch Vormischen) oder Nacheinander auf einer beliebigen Oberfläche appliziert. Durch Verdunsten des Lösungsmittels erfolgt Kristallisation der Matrix unter Einschluß der Analytverbindungen.

Ferner kann das Surface Preparation-Verfahren eingesetzt werden, wonach die Matrix bzw. das Matrixgemisch gelöst und ohne Analyt auf einer beliebigen Oberfläche appliziert wird. Durch Verdunsten des Lösungsmittels erfolgt die (Co-)Kristallisation der Matrixverbindung(en). Der gelöste Analyt wird auf die kristalline Matrix aufgetragen, wobei durch Auflösen lediglich oberflächennaher Matrixschichten bei Rekristallisation ein Einschluß der Analytverbindung in aufkonzentrierter Form erfolgt.

Das Sublimations-Verfahren entspricht dem Verfahren der Surface Preparation mit dem Unterschied, dass die Matrix nicht aus einer Lösung auskristallisiert, sondern unter Sublimation durch Abscheidung aus der Gasphase auf eine Oberfläche aufgetragen wird.

Schließlich ist ein sogenanntes Airbrush-Verfahren möglich, bei dem Matrix und Analyt in einem gemeinsamen Lösungsmittel(gemisch) gelöst und durch eine Sprühvorrichtung dispers verteilt werden (Aerosolbildung). Durch die große Oberfläche erfolgt eine rasche Verdunstung des Lösungsmittels unter Ausbildung kleiner Matrix-Analyt-Kristalle. Alternativ kann die Matrix auch ohne Analyt gelöst werden und auf zu untersuchende Oberflächen (zum Beispiel Gewebeabschnitte) gesprüht oder anderweitig appliziert werden.

Eine neuartige Anwendungsmöglichkeit stellt die Präparation der Matrix als ionische Flüssigkeit dar: zu diesem Zweck wird die gelöste Matrix mit äquimolarer Menge an kationisierbarer Base, wie Pyridin oder Diethylamin, versetzt und einrotiert, wodurch sich ein flüssiger ionischer Matrixfilm bildet, der mit der Analytlösung auf beliebigen Oberflächen appliziert werden kann.

Im folgenden wird unter einem "Verdau" ein an bestimmten Aminosäurepositionen enzymatisch geschnittenes Protein verstanden werden, wodurch viele kleinere Peptide entstehen.

Für die durchgeführten Untersuchungen wurde ein Voyager-DE STR Massenspektrometer von Applied Biosystems verwendet.

Das Massenspektrometer trennt unterschiedliche Ionenarten (Analytionen, Matrixionen) nach ihrem Masse/Ladungsverhältnis auf. Normalerweise tragen die Ionen in MALDI nur eine (positive oder negative) Ladung, so dass bei Ladung = 1 das Masse/Ladungsverhältnis gleich der Masse der Ionen ist. Die Abszisse der Spektren gibt das Massen/Ladungsverhältnis (und somit in annähernd allen Fällen die Masse) der Ionen wider, wobei die Einheit Dalton bzw. g/mol ist.

Stärkere Signale - die durch senkrechte Striche wiedergegeben sind, wobei die Höhe der Striche (Signale) mit der dieses Signal verursachenden Ionenspeziesmenge korreliert - werden einzeln mit ihrer Masse bzw. Masse/Ladungsverhältnis gekennzeichnet.

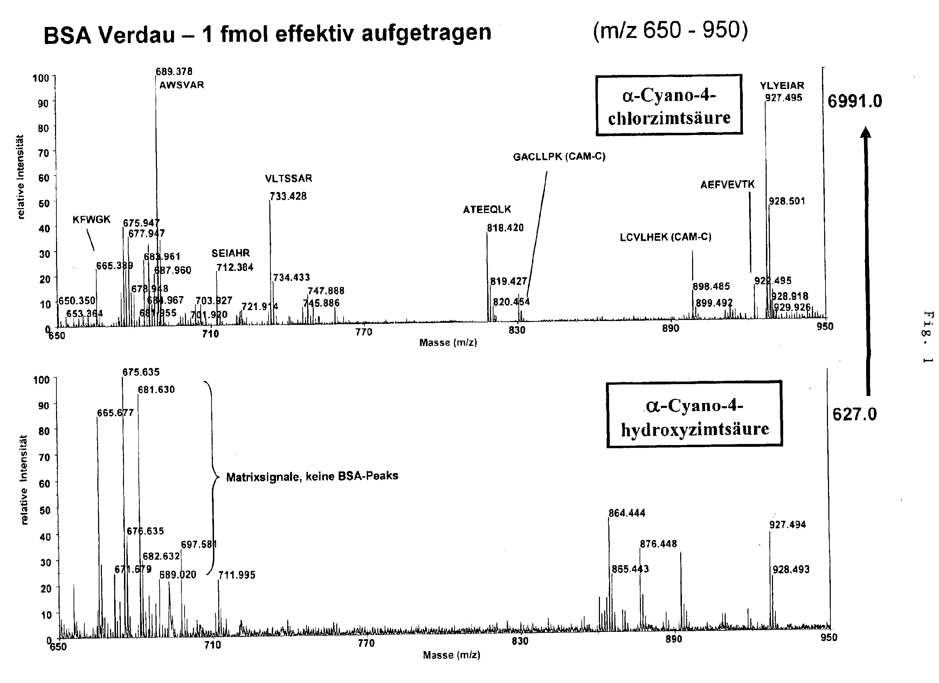

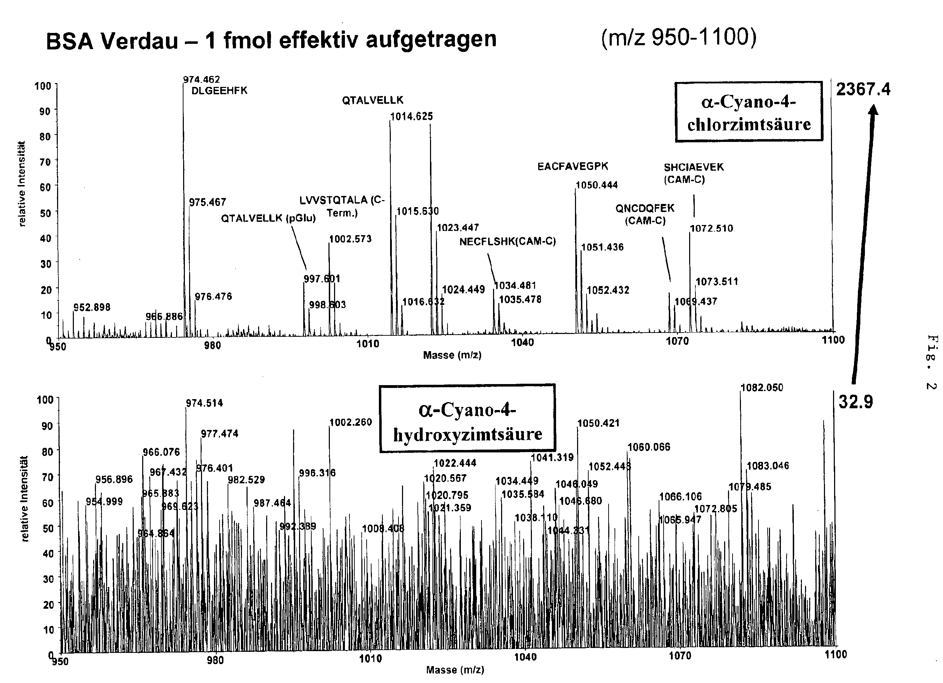

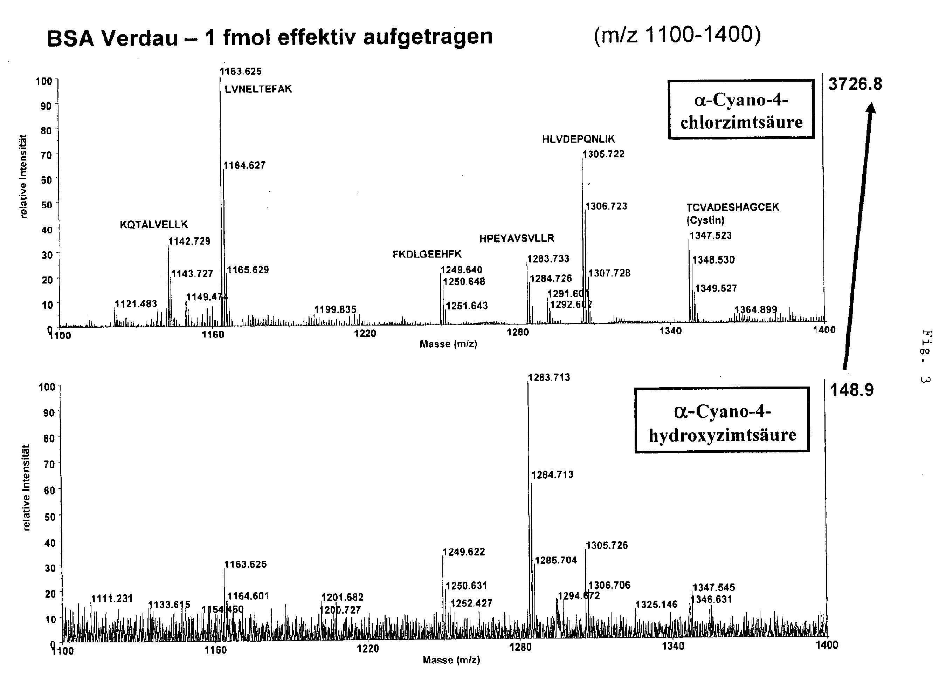

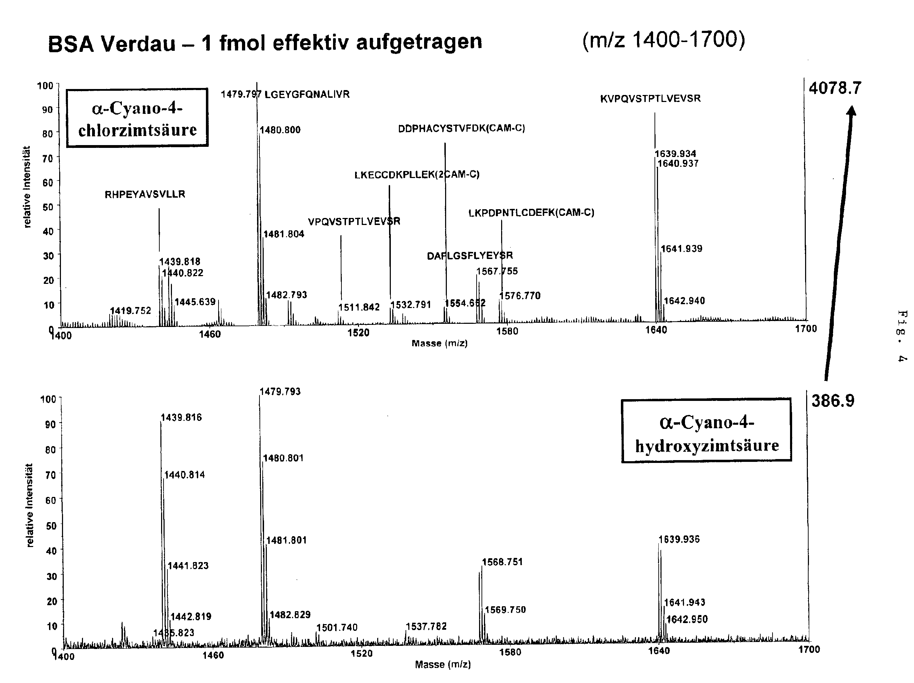

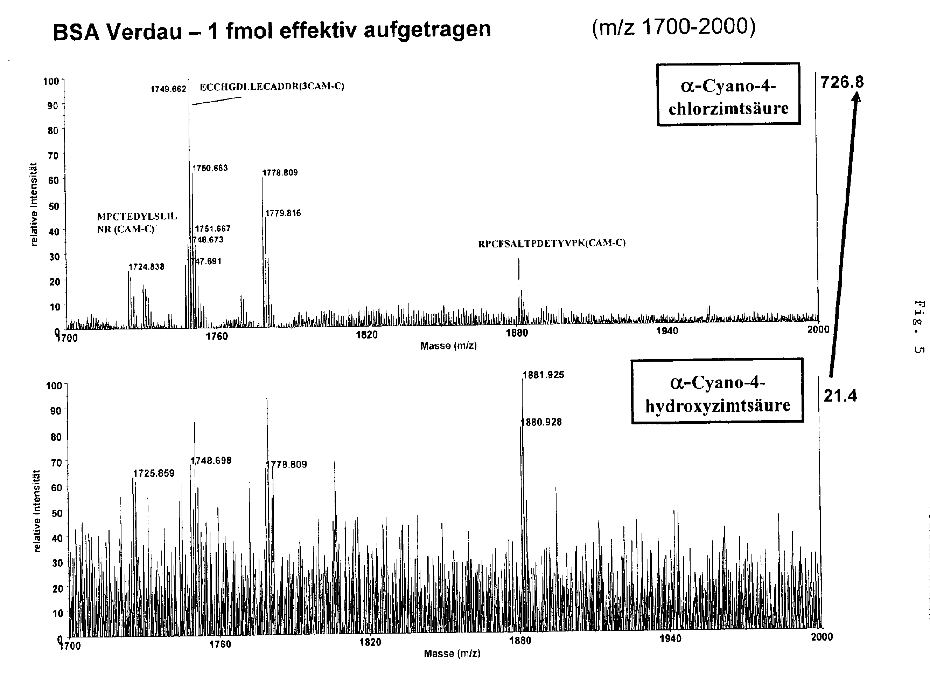

Die Ordinatenskala richtet sich nach dem stärksten Signal innerhalb des jeweiligen Spektrums und gibt den geräteabhängigen Absolutwert des stärksten Signals an. Alle anderen in dem Spektrum dargestellten Signale werden relativ zu dem stärksten Signal dargestellt (linke Ordinatenachse). Der rechte Ordinatenwert hat nur eine Aussagekraft bei Vergleichen mit weiteren Signalen innerhalb eines Spektrums oder anderen Spektren desselben Massenspektrometers. So geben die Zahlenwerte rechts oben in den Fig. 1-5 "BSA Verdau - 1fmol effektiv aufgetragen" die absoluten Intensitäten des jeweils stärksten Signals an (mit der Masse 689,378 im oberen Spektrum bei Verwendung der neuen Matrix α-Cyano-4-chlorzimtsäure in Fig. 1). Das Peptid mit der Masse m/z = 927,495 weist eine relative Intensität von 88% (abzulesen an der linken Ordinatenskala) bezüglich der Intensität des stärksten Ions bei m/z = 689,378 auf und damit eine Intensität von 6152 abs. Einheiten. Im unteren CHCA-Spektrum der bekannten Matrix α-Cyano-4-hydroxyzimtsäure hat dasselbe Signal bei m/z = 927,494 nur eine absolute Intensität von 253,1 Einheiten (berechnet nach dem stärksten Signal bei m/z = 675,635 mit 627,0 Einheiten, wobei das BSA-Peptid-Signal bei m/z = 927,494 eine relative Intensität von 40,37% aufweist). Die neue Matrix ermöglicht bei diesem exemplarischen Ion somit eine Zunahme der absoluten Intensität um einen Faktor von ungefähr 25, was bedeutet, dass dieses Peptid in Kombination mit der neuen Matrix deutlich mehr Ionen ausbildet und damit deutlich stärkere, leichter meßbare Signale bewirkt. Der Vorteil ist, dass schwache Signale, die bisher nicht detektierbar waren, nun aufgrund der Verstärkung gemessen werden können, was z.b. in Fig. 2, BSA Verdau - 1 fmol effektiv aufgetragen (950-1100) sichtbar wird: Die neue Matrix (Spektrum oben) ermöglicht eine Detektion von Analyten, die mit der alten (bisher besten) Matrix (Spektrum unten) nicht detektierbar waren. Zur genaueren Zuordnung wurden an die meisten Signale noch die Aminosäurezusammensetzungen der jeweiligen das Signal verursachenden Peptide hinzugefügt, wobei die Aminosäuremodifikation dem regulären 1-Buchstaben-Code entspricht. Mögliche Peptidmodifikationen sind in nachfolgenden Klammern angegeben (CAM-C: Carbamidomethylierung von Cystein, pGlu: pyroglutamin-Modifikation, C-term.: C-terminales Fragment). Die den Signalen nachfolgenden schwächeren Signale mit einem Massenzuwachs von 1 Da sind auf die ¹³C-Isotope der jeweiligen Peptide zurückzuführen. Die verbesserten Ergebnisse ergeben sich aus allen Fig. 1-5.

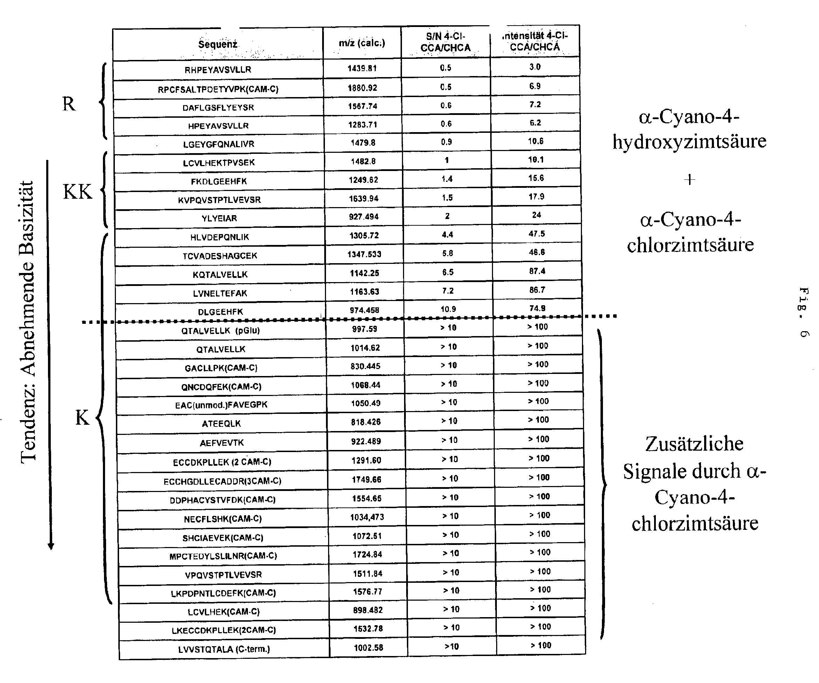

Fig. 6 listet alle detektierbaren Peptide des verwendeten BSA-Proteinverdaus auf. Die Menge der detektierbaren Peptide ist abhängig von der ursprünglich eingesetzten Proteinmenge, die in diesem Fall 1 fmol betrug, was eine sehr niedrige Menge darstellt. Die obere Hälfte zeigt die Peptide, die mit der bisherigen Matrix CHCA (Synonym HCCA oder α-Cyano-4-Hydroxyzimtsäure) detektierbar sind. Die untere Hälfte listet alle diejenigen Peptide auf, die zusätzlich zu der oberen Hälfte nur mit der neuen Matrix detektierbar sind. Daraus ist sofort erkennbar, dass ein immenser Informationsvorteil geschaffen wird. Die erste Spalte listet die Aminosäuresequenz der jeweiligen Peptide auf, die zweite Spalte deren Masse. In der dritten Spalte wird das S/N (Signal to Noise, Signal zu Rauschen) - Verhältnis angegeben, das eine Aussage über die Intensität und Detektierbarkeit eines Signals macht: Das gesamte Spektrum weist aufgrund verschiedener Umstände ein leichtes Rauschen auf, das immer auftritt, auch wenn keine Analyte enthalten sind. Signale müssen zur Detektion höhere Intensitäten als das Rauschen aufweisen, andernfalls sie nicht detektierbar sind. Das S/N-Verhältnis wird vom Computer berechnet und gibt an, wie viel mal stärker ein Signal relativ zu dem Hintergrund-rauschen ist. Damit gilt: Je höher, desto leichter ist das Signal zu erkennen und zu detektieren. In der dritten Spalte ist nun das S/N-Verhältnis von der neuen Matrix relativ zu der alten Matrix aufgenommen, Werte > 1 geben somit an, dass diese Signale stärker sind und somit leichter detektiert werden können. In der vierten Spalte ist das Verhältnis der abs. Intensitäten angegeben, erneut gilt, je größer, desto leichter ist ein Signal als solches zu erkennen, es ist klar zu erkennen, dass die neue Matrix deutlich stärkere Signale als die alte Matrix erzeugt.

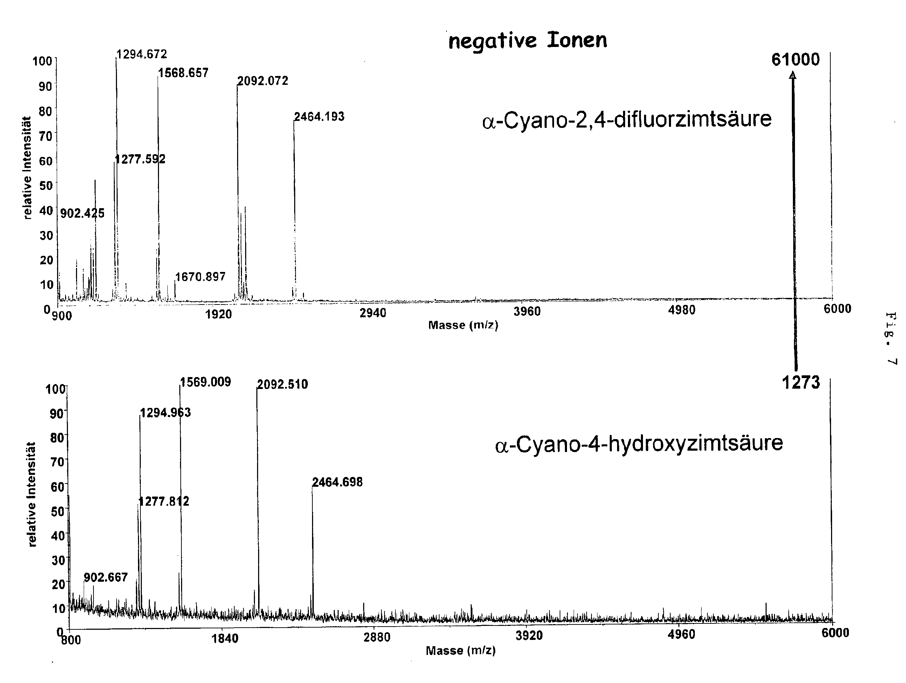

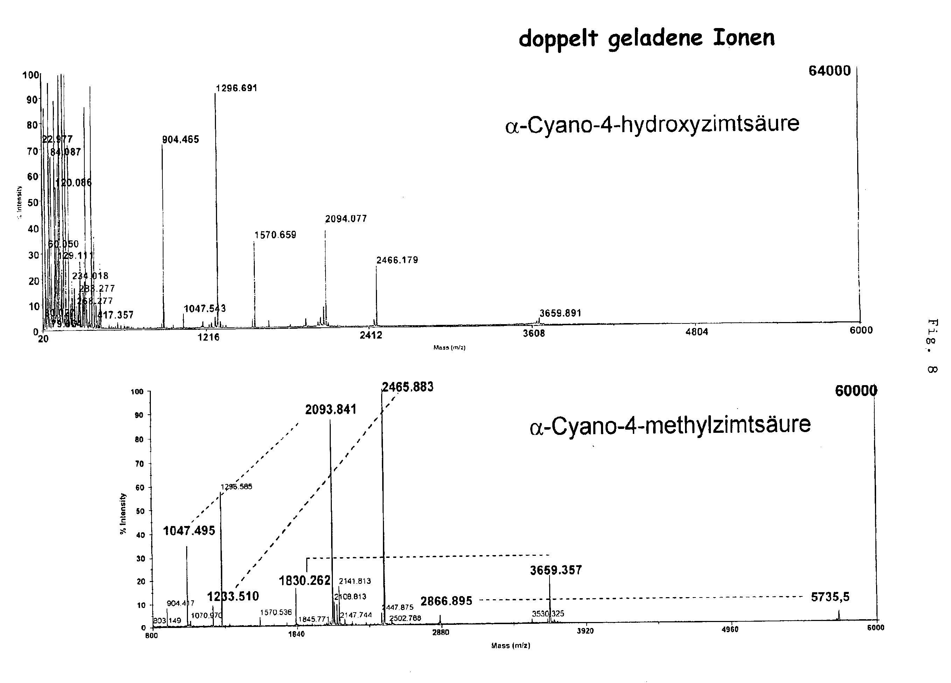

Die für Figuren 1-5 diskutierten und gegenüber dem Stand der Technik deutlich verbesserten Ergebnisse zeigen sich auch bei den Massenspektren der Figuren 7 und 8 für negative bzw. doppelt geladene Ionen.

Die in der vorstehenden Beschreibung, in den Ansprüchen und in den Zeichnungen offenbarten Merkmale der Erfindung können sowohl einzeln als auch in Kombination zur Verwirklichung der Erfindung in ihren unterschiedlichsten Ausführungsformen wesentlich sein.

## Patentansprüche

1. Verwendung von Cyanozimtsäurederivaten der allgemeinen Formel : 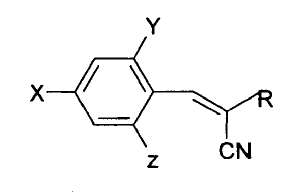 wobei X ausgewählt wird aus Cl, F, Br, I und substituiertem und unsubstituiertem C₁-C₁₀-Alkyl; Y und Z unabhängig ausgewählt werden aus H, Cl, F, Br, I und substituiertem und unsubstituiertem C₁-C₁₀-Alkyl; und R ausgewählt wird aus COOH, CONH₂, SO₃H und COOR' mit R' = C₁-C₁₀-Alkyl
als Matrix für eine MALDI-Massenspektrometrie eines Analyten.

2. Verwendung nach Anspruch 1, **dadurch gekennzeichnet, dass** X ausgewählt wird aus F, Cl, Methyl oder C₂-C₁₀-Alkyl und Y und Z unabhängig ausgewählt werden aus H, F und Cl.

3. Verwendung nach Anspruch 1 oder 2, **dadurch gekennzeichnet, dass** das Cyanozimtsäurederivat 2,4-Difluor-cyanozimtsäure, 4-Chlor-cyanozimtsäure oder 4-Methyl-cyanozimtsäure ist.

4. Verwendung nach einem der vorangehenden Ansprüche, **dadurch gekennzeichnet, dass** 4-Chlor-cyanozimtsäure die Matrix zur MALDI-Massenspektrometrie positiver Ionen ist.

5. Verwendung nach einem der vorangehenden Ansprüche, **dadurch gekennzeichnet, dass** 2,4-Difluor-cyanozimtsäure, 4-Chlor-cyanozimtsäure und 4-Methyl-cyanozimtsäure Matrizes zur MALDI-Massenspektrometrie negativer Ionen sind.

6. Verwendung nach einem der vorangehenden Ansprüche 1 bis 4, **dadurch gekennzeichnet, dass** 2,4-Difluor-cyanozimtsäure die Matrix zur MALDI-Massenspektrometrie negativer und positiver Ionen ist.

7. Verwendung nach einem der vorangehenden Ansprüche, **dadurch gekennzeichnet, dass** 4-Methyl-cyanozimtsäure und 4-Chlor-cyanozimtsäure Matrizes zur MALDI-Massenspektrometrie zweifach oder höhergeladener Ionen sind.

8. Verwendung nach einem der vorangehenden Ansprüche, **dadurch gekennzeichnet, dass** das Analyt ein verdautes Protein oder Peptid ist.

9. Verwendung nach einem der vorangehenden Ansprüche, **dadurch gekennzeichnet, dass** die Matrix mit dem Analyten vermischt wird.

10. Verwendung nach einem der vorangehenden Ansprüche, **dadurch gekennzeichnet, dass** Mischungen unterschiedlicher unter obige allgemeine Formel fallende Cyanozimtsäure-Derivate oder eine Mischung zumindest eines unter obige allgemeine Formel fallenden Cyanozimtsäure-Derivats mit einer weiteren Matrix eingesetzt werden bzw. wird.

11. Verwendung nach Anspruch 10, **dadurch gekennzeichnet, dass** die weitere Matrix ausgewählt wird aus α-Cyano-4-hydroxyzimtsäure, 2,5-Dihydroxybenzoesäure oder Sinapin- oder Ferulasäure.

## Claims

1. Use of cyanocinnamic acid of the general formule: 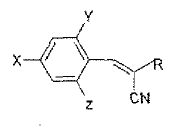 wherein X is selected from Cl, F, Br, I, and substituted and unsubstituted C₁-C₁₀-alkyl; Y and Z are independently selected from H, Cl, F, Br, I, and substituted and unsubstituted C₁-C₁₀-alkyl; and R are selected from COOH, CONH₂, SO₃H and COOR' with R' = C₁-C₁₀-alkyl
as matrices for a MALDI- mass spectrometry observation of an analyte.

2. Use according to claim 1, **characterized in that** X is selected from F, Cl, methyl or C₂-C₁₀-alkyl and Y and Z are independently selected from H, F, and Cl.

3. Use according to claim 1 or 2, **characterized in that** the cyanocinnamic acid derivative is 2,4-difluoro-cyanocinnamic acid, 4-chloro-cyanocinnamic acid, or 4-methyl-cyanocinnamic acid.

4. Use according to one of the preceding claims, **characterized in that** 4-chloro-cyanocinnamic acid is the matrix for the MALDI mass spectrometry observation of positive ions.

5. Use according to one of the preceding claims, **characterized in that** 2,4-difluoro-cyanocinnamic acid, 4-chloro-cyanocinnamic acid, and 4-methyl-cyanocinnamic acid are matrices for the MALDI mass spectrometry observation of negative ions.

6. Use according to one of the preceding claims 1 to 4, **characterized in that** 2,4-difluoro-cyanocinnamic acid is the matrix for the MALDI mass spectrometry observation of negative and positive ions.

7. Use according to one of the preceding claims, **characterized in that** 4-methyl-cyanocinnamic acid and 4-chloro-cyanocinnamic acid are matrices for the MALDI mass spectrometry observation of twofold or higher charged ions.

8. Use according to one of the preceding claims, **characterized in that** analyze is a digested protein or peptide.

9. Use according to one of the preceding claims, **characterized in that** the matrix is mixed with the analyte.

10. Use according to one of the preceding claims, **characterized in that** mixtures of different cyanocinnamic acid derivatives falling under the general formula given heretofore, or a mixture of at least one cyanocinnamic acid derivative falling under the general formula given heretofore are or is added to a further matrix.

11. Use according to claim 10, **characterized in that** the further matrix is selected from a-cyano-4-hydroxycyanocinnamic acid, 2,5-dihydroxybenzoic acid, or sinapic acid or ferluic acid.

## Revendications

1. Utilisation de dérivés d'acide cyanocinnamique de formule générale : 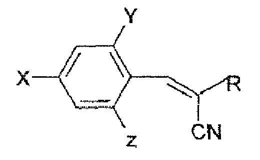 dans laquelle X est choisi parmi Cl, F, Br, I et un groupe alkyle en Cl à C₁₀ substitué et non substitué ; Y et Z sont choisis indépendamment parmi H, Cl, F, Br, I et un groupe alkyle en C₁ à C₁₀ substitué et non substitué ; et R est choisi parmi COOH, CONH₂, SO₃H et COOR' avec R' = alkyle en C₁ à C₁₀
comme matrice pour une spectrométrie de masse MALDI d'un analyte.

2. Utilisation selon la revendication 1, **caractérisée en ce que** X est choisi parmi F, Cl, méthyle ou un groupe alkyle en C₂ à C₁₀ et Y et Z sont choisis indépendamment parmi H, F et Cl.

3. Utilisation selon la revendication 1 ou 2, **caractérisée en ce que** le dérivé d'acide cyanocinnamique est l'acide 2,4-difluoro-cyanocinnamique, l'acide 4-chlorocyanocinnamique ou l'acide 4-méthylcyanocinnamique.

4. Utilisation selon une des revendications précédentes, **caractérisée en ce que** l'acide 4-chlorocyanocinnamique est la matrice pour la spectrométrie de masse MALDI des ions positifs.

5. Utilisation selon une des revendications précédentes, **caractérisée en ce que** l'acide 2,4-difluorocyanocinnamique, l'acide 4-chlorocyanocinnamique et l'acide 4-méthylcyanocinnamique sont des matrices pour la spectrométrie de masse MALDI des ions négatifs.

6. Utilisation selon une des revendications précédentes 1 à 4, **caractérisée en ce que** l'acide 2,4-difluorocyanocinnamique est la matrice pour la spectrométrie de masse MALDI des ions négatifs et positifs.

7. Utilisation selon une des revendications précédentes, **caractérisée en ce que** l'acide 4-méthylcyanocinnamique et l'acide 4-chlore-cyanocinnamique sont des matrices pour la spectrométrie de masse MALDI d'ions chargés deux fois ou plus.

8. Utilisation selon une des revendications précédentes, **caractérisée en ce que** l'analyte est une protéine ou un peptide digéré(e).

9. Utilisation selon une des revendications précédentes, **caractérisée en ce que** la matrice est mélangée avec l'analyte.

10. Utilisation selon une des revendications précédentes, **caractérisée en ce que** des mélanges des différents dérivés d'acide cyanocinnamique englobés par la formule générale ci-dessus ou un mélange d'au moins un dérivé d'acide cyanocinnamique englobé par la formule générale ci-dessus sont ou est mis en oeuvre avec une autre matrice.

11. Utilisation selon la revendication 10, **caractérisée en ce que** l'autre matrice est choisie parmi l'acide α-cyano-4-hydroxycinnamique, l'acide 2,5-dihydroxybenzoïque ou l'acide sinapique ou férulique.
